Europäisches Patentamt

European Patent Office

Office européen des brevets

(11) Publication number: **0 129 947**
**B1**

## EUROPEAN PATENT SPECIFICATION

(45) Date of publication of patent specification: **14.09.88**

(51) Int. Cl.⁴: **C 07 J 1/00, C 07 J 41/00,
A 61 K 31/565**

(21) Application number: **84300611.5**

(22) Date of filing: **31.01.84**

(54) Contraceptive compositions based on esters of levo-norgestrel.

(30) Priority: **19.05.83 GB 8313921**

(43) Date of publication of application:
**02.01.85 Bulletin 85/01**

(45) Publication of the grant of the patent:
**14.09.88 Bulletin 88/37**

(84) Designated Contracting States:
**AT BE CH DE FR GB IT LI LU NL SE**

(56) References cited:
**FR-A-2 318 645**

**STEROIDS, vol. 41, no. 3, March 1983, pages
349-359, Holden-Day, San Francisco (US). A.
SHAFIEE et al.: "Long-acting contracetive
agents: aliphatic and alicyclic carboxylic esters
of levonorgestrel"**

(73) Proprietor: **WORLD HEALTH ORGANISATION
CH-1211 Geneva 27 (CH)**

(72) Inventor: **Archer, Sidney Department of
Chemistry
Renssalaer Polytecnic Institute
Troy New York 12181 (US)**
Inventor: **Diczfalusy, Egon Swedish Medical
Research Council Karolinska Sjukhuset
S-104 01 Stockholm 60 (SE)**
Inventor: **Fried, Josef Department of Chemistry
The University of Chicago 5735 South Ellis
Avenue
Chicago Illinois 60637 (US)**
Inventor: **Benagiano, Guiseppe
Universita di Roma Policlinico Umberto 1
I-00161 Rome (IT)**
Inventor: **Crabbe, Pierre
11 Rue de l'Abbe Gregoire
F-75006 Paris (FR)**
Inventor: **Djerassi, Carl
Department of Chemistry Stanford University
Stanford California 94305 (US)**

(74) Representative: **Arthur, Bryan Edward et al
Withers & Rogers 4 Dyer's Buildings Holborn
London EC1N 2JT (GB)**

(56) References cited:

**STEROIDS, vol. 41, no. 3, March 1983, pages 419-439, Holden-Day, San Francisco (US). G. BIALY et al.: "Long-acting contraceptive agents: structure activity relationships in a series of norethisterone and levonorgestrel esters"**

**BULLETIN DES SOCIETES CHIMIQUES BELGES, vol. 92, no. 3, March 1983, Brussels (BE), pages 275-287, PIERRE CRABBE et al.: "Long-acting contracetive agents: X-ray study of levonorgestrel esters"**

**CHEMICAL ABSTRACTS, vol. 97, no. 21, 22 November 1982, page 860, abstract 182724x (COLUMBUS, OHIO, US). R. VLAKHOV et al.: "Synthesis of some esters of norethisterone and d-levonorgestrel as possible fertility regulators"**

## Description

This invention relates to injectable therapeutic compositions based on certain esters of levo-norgestrel which have valuable long-acting contraceptive properties.

The pharmacological effects of steroids such as levo-norgestrel, progesterone, nortestosterone and norethisterone have been known for very many years. However, their effects as contraceptive agents are relatively short lasting with the consequence that injections at frequent intervals would be required to maintain fertility control.

Attempts have been made to extend the effective term of contraceptive activity by esterifying the steroids, a very desirable term being three months or more. However, to date, only two injectable contraceptive preparations have been made available. These are depot-medroxy-progesterone acetate (DMPA), which initially has to be injected at 2-monthly intervals, and norethisterone enanthate. Both of these suffer from several disadvantages one of them being that although they were developed in the 1960's they have still not been approved by the US Food and Drug Administration.

There is a great need, particularly in developing countries, for an injectable contraceptive which is safe and is effective for a longer period.

This need is stressed in patent specification FR—A—2318645 wherein there are described certain esters in the d series of the 3-oximes of d-norgestrel and a method of suppressing fertility of female animals by administration of such esters. The esters are said to be suitable as long-acting compounds for the suppression of fertility when administered parenterally.

With this need in mind, new derivatives of synthetic steroids known to be efficient and "safe" as contraceptive agents have been selected and synthesised. Synthesised oximes of 17-esters of levo-norgestrel with acids of varying chain lengths, nature and degree of unsaturation, (double and/or triple bonds, dienes, enynes, allenes), ring size (cyclopropane to cyclohexane, with or without substitution) were subjected to a biological screening programme designed to uncover new and effective sustained-release injectable contraceptives, the compounds being tested as injectable oily solutions or aqueous suspensions. The initial test was to determine the duration of suppression of estrus in female rats, with DMPA and norethisterone enanthate being used as comparison standards. The most promising compounds were then tested in primates.

In general, the tests indicated a lack of correlation between structure and activity, but it was found that of the compounds screened two showed outstanding properties when injected as a micro crystalline suspension in an aqueous medium. The two compounds were levo-norgestrel cyclopentylcarboxylate 3-oxime and cyclohexylcarboxylate 3-oxime.

It was noted that the duration of action of these two longer acting compounds was also dependent on the nature of the pharmaceutical composition into which they were formulated, even better results being achieved when the compounds were injected as an aqueous suspension of crystals of a particular particle size range. Further, the longer term effectiveness was achieved even with doses smaller than the normal doses of DMPA.

The present invention therefore provides an ester of levo-norgestrel and an aliphatic acid characterised in that the ester is:

levo-norgestrel cyclopentylcarboxylate 3-oxime
levo-norgestrel cyclohexylcarboxylate 3-oxime.

The present invention also provides injectable contraceptive compositions which are suspensions in a pharmaceutically acceptable aqueous medium of one of these selected esters, preferably of micro-crystals of particle size in the range 3 to 10 μ expressed as the 50% cumulative oversize in the Coulter distribution curve.

Preferably, the composition is in a unit dosage form containing 1 to 50 mg of the levo-norgestrel ester.

In general, it is desirable that any administration of steroids is in the smallest dose effective for the purpose and it is a feature of this invention that the compositions if injected at intervals appropriate for DMPA can contain a smaller amount of the steroid, and further, that the dose may still be smaller even when the injections are at longer intervals of say, 3 months.

The invention therefore makes possible a method of contraception wherein there is injected into a human female a dose of an above-mentioned composition containing micro-crystals of one of the above selected levo-norgestrel esters of particle size in the range 3 to 10 μ, the dose containing from 1 to 50 mg of the ester.

Preferably, the method of contraception is a continuous one, the injections being given at intervals of from two to six months, though injections can be given at one month intervals if necessary. A suitable dose at one month intervals is from 1 to 6 mg, and at six month intervals is from 20 to 50 mg.

By levo-norgestrel is meant D -(−)13β - ethyl - 17α - ethynyl - 17β - hydroxygon - 4 - en - 3 - one.

The oximes are prepared by reacting the appropriate ester with hydroxylamine hydrochloride.

For example, levo-norgestrel cyclopentylcarboxylate 3-oxime was prepared as follows:

Levo-norgestrel 17β-cyclopentyl carboxylate (588 mg) was dissolved in pyridine (6 ml) and hydroxylamine-hydrochloride (550 mg) added and the mixture heated at 100°C for 7 minutes and cooled in an ice-bath. Hydrochloric acid (1*N*, 45 ml) was added dropwise and the mixture extracted with ethyl acetate. The ethyl acetate extract was washed with 1*N* HCl, water, saturated sodium bicarbonate solution, water

3

and brine and dried over sodium sulphate. Evaporation of the solvent and crystallisation of the residue from ethanol-water and drying *in vacuo* gave the oxime (593 mg), mp 172.5—174°C; HPLC (µPorasil, heptane: isopropanol 96:4, 2 ml/min, U.V. 254 nm, *anti* isomer:*syn* isomer 53:47).

A similar preparation of levo-norgestrel 17β-cyclohexyl carboxylate 3-oxime gave the oxime (2.2 g) mp 167—169°C as a mixture of the *anti* and *syn* isomers (53:47 by HPLC -µPorasil column, heptane:isopropanol 96:4).

The microcrystalline suspensions of the steroid esters were made by milling the appropriate amount of the ester in an aqueous medium consisting of:

| | |
|---|---|
| Benzyl alcohol | 1.000% w/v |
| Sodium carboxymethyl cellulose | 0.500% w/v |
| Disodium hydrogen phosphate dihydrate | 1.495% w/v |
| Polysorbate 80 | 0.200% w/v |
| Water for injection to | 100.000% |

The milling was carried out in a Glen Creston M270 micronising ball mill equipped with an agate container and ball. A milling period of up to an hour was required to reduce the particles of ester to the required size.

The bioassay used to determine the duration of action of the contraceptive compositions involved measuring the suppression of estrus in female rats. The compositions were used at four dosage levels, i.e. 2, 4, 8 and 16 mg/rat.

The long-acting properties of each formulation were determined in estrus suppression assay employing virgin, mature (180—200 g) cycling rats of the Sprague-Dawley strain purchased from Charles River Laboratories, USA. Upon receipt the animals were smeared daily for cyclicity and only those animals showing two consecutive normal cycles were used. Each animal was injected subcutaneously with 0.8 ml of the test preparation on the same day regardless of the stage of the cycle. Each compound was initially tested on ten rats. A series of initial tests with injections of 16 mg/animal were followed by studies at dose levels of 8, 4 and 2 mg/animal where necessary. An aqueous microcrystalline suspension of medroxyprogesterone acetate was used as standard. Daily smears were taken starting on the day after injection and continued until such time that cornification of vaginal epithelium was observed and cycling was re-established. Duration of cornification suppression is expressed as the total number of days minus two. The appearance of cornification was not always followed by the return of normal estrus cycles. The majority of experiments were terminated at 91 days post injection even though some of the animals had not exhibited cornified smears.

A series of tests using formulations based on leveo-norgestrel ester oximes produced unexpected results.

Most were tested as aqueous microcrystalline suspensions of the oxime though in the cases of the oximes of levo-norgestrel butanoate, levo-norgestrel isobutanoate, levo-norgestrel cyclopropylcarboxylate and levo-norgestrel itself, the ester oxime could not be formulated as an aqueous microcrystalline suspension and had to be formulated as a solution in ethyl oleate. Differences in activity were observed among formulations having different particle size distributions; microcrystalline suspensions being more active than the coarser preparations.

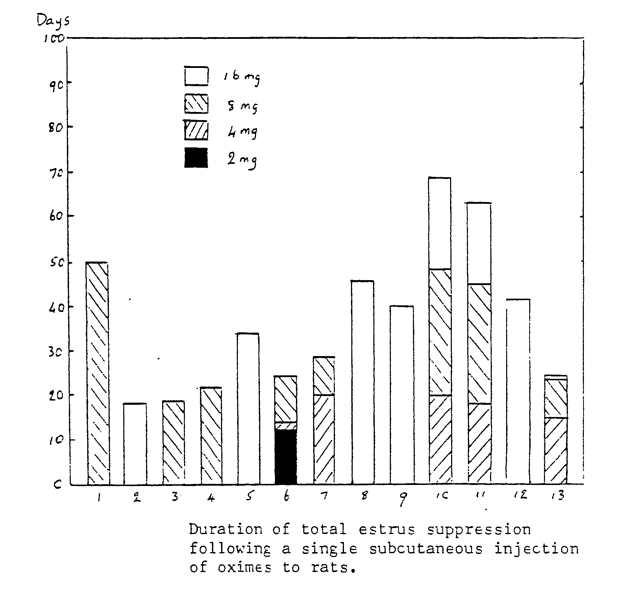

The results are shown in the accompanying drawing in which formulation 1 was based on depot - medroxy - progesterone - acetate and the following formulations were based on oximes of

2. levo-norgestrel
3. levo-norgestrel butanoate
4. levo-norgestrel isobutanoate
5. levo-norgestrel isovalerate
6. levo-norgestrel cyclopropylcarboxylate
7. levo-norgestrel cyclobutylcarboxylate
8. levo-norgestrel (3'-methyl) cyclohexylcarboxylate
9. levo-norgestrel (3'-ethyl) cyclohexylcarboxylate
10. levo-norgestrel cyclopentylcarboxylate
11. levo-norgestrel cyclohexylcarboxylate
12. levo-norgestrel (4'-methyl) cyclohexylcarboxylate
13. levo-norgestrel acetate

Although the cyclopentylcarboxylate and cyclohexylcarboxylate esters are relatively ineffective even at 16 mg dose, it was found that their oximes at an 8 mg dose were comparable with DMPA. The two oximes also were surprisingly more effective than closely related ester oximes such as levo-norgestrel cyclobutylcarboxylate 3-oxime and levo - norgestrel - (4' - methyl)cyclohexylcarboxylate 3-oxime.

Again, it was noted that the cyclopentyl and cyclohexyl carboxylate 3-oximes gave better results when formulated as an aqueous microcrystalline suspension of particle size 3 to 10 µ expressed as the 50% cumulative oversize in the Coulter distribution curve.

It has been observed that progestogens themselves give rise to some endometrial bleeding between injections, but the tests on the preferred oximes indicate that they have a reduced tendency to cause such bleeding and so are more attractive.

**Claims**

1. An ester of levo-norgestrel and an aliphatic acid characterised in that the ester is levo-norgestrel cyclopentylcarboxylate 3-oxime.

2. An ester of levo-norgestrel and an aliphatic acid characterised in that the ester is levo-norgestrel cyclohexylcarboxylate 3-oxime.

3. An injectable contraceptive composition which is a suspension of a levo-norgestrel ester in a pharmaceutically acceptable aqueous medium characterised in that the ester is one claimed in claim 1 or claim 2.

4. A composition as claimed in claim 3 characterised in that the ester is suspended in the form of microcrystals of particle size in the range 3 to 10 μ expressed as the 50% cumulative oversize in the Coulter distribution curve.

5. A composition as claimed in claim 3 or claim 4 characterised in that it is in a unit dosage form containing from 1 to 50 mg of the ester.

**Patentansprüche**

1. Ester von Levo-Norgestrel und eine aliphatische Säure, dadurch gekennzeichnet, daß der Ester Levo - Norgestrel - Cyclopentylcarboxylat - 3 - Oxim ist.

2. Ester von Levo-Norgestrel und eine aliphatische Säure, dadurch gekennzeichnet, daß der Ester Levo - Norgestrel - Cyclohexylcarboxylat - 3 - Oxim ist.

3. Injizierbare kontrazeptive Zuammensetzung, die eine Suspension von Levo-Norgestrelester in einem pharmazeutisch annehmbaren wässrigen Medium ist, dadurch gekennzeichnet, daß der Ester ein solcher ist, wie in Anspruch 1 oder 2 beansprucht.

4. Zusammensetzung nach Anspruch 3, dadurch gekennzeichnet, daß der Ester in der Form von Mikrokristallen einer Teilchengröße im Bereich von 3 bis 10 μ, ausgedrückt als die 50%-kumulative Übergröße in der Coulter-Verteilungskurve suspendiert ist.

5. Zusammensetzung nach Anspruch 3 oder 4, dadurch gekennzeichnet, daß sie in Form einer Einheitsdosis vorliegt, die 1 bis 50 mg des Esters enthält.

**Revendications**

1. Un ester de levo-norgestrel et d'un acide aliphatique, caractérisé en ce que l'ester consiste en le cyclo - pentylcarboxylate - 3 - oxime de levo-norgestrel.

2. Un ester de levo-norgestrel et d'un acide aliphatique, caractérisé en ce que l'ester consiste en le cyclo - pexylcarboxylate - 3 - oxime de levo-norgestrel.

3. Une composition contraceptive injectable consistant en une suspension d'un ester de levo-norgestrel dans un milieu aqueux pharmaceutiquement acceptable, caractérisé en ce que l'ester est l'un des esters revendiqués dans la revendication 1 et dans la revendication 2.

4. Une composition telle que revendiquée dans la revendication 3, caractérisée en ce que l'ester est sous forme de suspension de micro-cristaux dont les dimensions particulaires, exprimées par la méthode du surdimensionnement cumulatif de 50% dans la courbe de distribution de Coulter, sont comprises entre 3 et 10 μ.

5. Une composition telle que revendiquée dans la revendication 3 ou dans la revendication 4, caractérisée en ce qu'elle est sous forme d'une dose unitaire contenant de 1 à 50 mg dudit ester.

Duration of total estrus suppression
following a single subcutaneous injection
of oximes to rats.